# EUROPÄISCHE PATENTSCHRIFT

(11) **EP 3 741 290 B1**
(45) Veröffentlichungstag und Bekanntmachung des Hinweises auf die Patenterteilung: **15.12.2021**
(21) Anmeldenummer: 20173659.2
(22) Anmeldetag: 08.05.2020
(51) Int. Cl.: A61B 5/00

(54) **VORRICHTUNG ZUR ABBILDUNGSERZEUGUNG VON HAUTLÄSIONEN**
DEVICE FOR GENERATING IMAGES OF SKIN LESIONS
DISPOSITIF D'IMAGERIE DE LÉSIONS CUTANÉES

(30) Priorität: 20.05.2019 DE 102019113283
(43) Veröffentlichungstag der Anmeldung: 25.11.2020
(73) Patentinhaber: Fotofinder Systems GmbH, 84364 Bad Birnbach (DE)
(72) Erfinder: Fuchs, Tobias, 94065 Waldkirchen (DE); Weber, Sebastian, 84364 Bad Birnach (DE)
(74) Vertreter: advotec.

(56) Entgegenhaltungen:
- EP-A1- 0 826 335
- WO-A2-2007/064329
- US-A1- 2010 214 562
- NICHOLAS MACKINNON ET AL: "In vivo skin chromophore mapping using a multimode imaging dermoscope (SkinSpec?)", SPIE - INTERNATIONAL SOCIETY FOR OPTICAL ENGINEERING. PROCEEDINGS, Bd. 8587, 22. Februar 2013 (2013-02-22), Seite 85870U, XP055722897, US ISSN: 0277-786X, DOI: 10.1117/12.2005587 ISBN: 978-1-5106-3549-4

## Beschreibung

Die Erfindung betrifft eine Vorrichtung und ein Verfahren zur Erzeugung von Abbildungen zur Unterstützung bei der Charakterisierung von Hautläsionen eines menschlichen Körpers.

Ein aus dem Stand der Technik bekanntes Verfahren zur Erfassung von Hautläsionen, d.h. von Hautveränderungen und Hautschädigungen, ist die Dermatoskopie bzw. die Auflichtmikroskopie, ein nichtinvasives Untersuchungsverfahren bei dem die zu untersuchenden Hautbereiche mit einem Mikroskop unter Beleuchtung mit polarisiertem Licht analysiert werden können. Eine Einschätzung bzw. Diagnose erfolgt hierbei durch den behandelnden Arzt mittels visueller Begutachtung. Diese kann dann mit einer zusätzlichen histologischen Untersuchung des Hautbereichs bestätigt werden, für welche jedoch ein chirurgischer Eingriff zur Entnahme einer Gewebeprobe notwendig ist.

Zur Verbesserung dieser rein visuellen Begutachtung durch einen behandelnden Arzt wurden zudem Spektralbildtechnologien und hierauf basierende bildgebende Vorrichtungen entwickelt, um die Oberflächenschichten eines zu untersuchenden Hautbereichs hinsichtlich ihrer Spektraleigenschaften zu untersuchen, welche durch Chromophoren wie beispielsweise Melanin und Hämoglobin charakterisiert werden und welche Rückschlüsse hinsichtlich einer Charakterisierung von Hautläsionen erlauben.

US 4,515,165 offenbart beispielsweise eine Vorrichtung und ein Verfahren zum Nachweis von Tumoren in Gewebe unter Verwendung der Transmission oder Reflexion von Licht mit einer Wellenlänge im Bereich von 400 bis etwa 700 Nanometern und Infrarotlicht, woraus ein multispektrales Bild des Gewebes erstellt wird.

EP 1 011 446 A1 offenbart ein System und Verfahren zur Charakterisierung einer Läsion als gutartig oder bösartig basierend auf einer multispektralen Bildgebung des Hautbereichs beleuchtet durch eine Lichtquelle unter Verwendung unterschiedlicher Filter.

US 2015/3200316 A1 offenbart eine Vorrichtung zur multispektralen Bildgebung, wobei eine Hautläsion mit unterschiedlichen Wellenlängen im sichtbaren und infraroten Spektrum durchleuchtet wird, um multispektrale Bilder in einem dreidimensionalen virtuellen Raum abzubilden. Hierbei werden zu Analysezwecken 3D-Verleitungen von vordefinierten Parametern bereitgestellt, um charakteristische Eigenschaften wie Melanin, Hämoglobin und Desoxyhämoglobin einer Hautläsion darzustellen.

US 2010/214562 offenbart eine Sonde, die eine integrierte konfokale Reflexionsbildgebung, konfokale Raman-Spektroskopie und eine grobe räumliche Bildgebung zur nichtinvasiven Bewertung eines lebenden Körpers verwendet, aufweisend ein Gehäuse mit einem ersten und einem gegenüberliegenden zweiten Ende, sowie einem ersten, zweiten und dritten optischen Anschluss. Das Gehäuse und der dritte optische Anschluss befinden sich am zweiten Ende des Gehäuses, so dass der erste und der dritte optische Anschluss einen ersten optischen Pfad zwischen ihnen definieren und der zweite und dritte optische Anschluss jeweils einen zweiten optischen Pfad zwischen ihnen definieren, wobei jeder der ersten und zweiten optischen Pfade einen ersten Abschnitt und einen zweiten Abschnitt aufweist, und wobei die zweiten Abschnitte des ersten und zweiten optischen Pfades im Wesentlichen überlappt sind und proximal zum dritten optischen Anschluss liegen.

WO 2007/064329 A2 offenbart ein optisches hyperspektrales Bildgebungsverfahren und eine zugehörige Vorrichtung, um eine hohe Signalempfindlichkeit für die Implementierung verschiedener optischer Bildgebungsansätze bereitzustellen. Die Vorrichtung weist dabei Mikroskopobjektive auf, um eine außeraxiale Beleuchtung von zu untersuchendem Gewebe zu ermöglichen, wobei eine Anregung bei jeder optischen Wellenlänge ermöglicht wird.

MacKinnon, Nicholas et al: "In vivo skin chromophore mapping using a multimode imaging dermoscope", ISBN: 978-1-5106-3549-4 offenbart ein Multimode-Dermoskop zur Melanom-Früherkennung, welches Fluoreszenz-, Polarisations- und Hyperspektralbildgebung kombiniert.

Die bekannten Vorrichtungen oder Systeme weisen den Nachteil auf, dass die zur Erfassung der spektralen Informationen notwendigen Vorrichtungen oft sehr sperrig oder unhandlich sind. Zudem sind die erzeugten Abbildungen zur zuverlässigen Charakterisierung entweder nicht aussagekräftig genug oder es wird eine Vielzahl von Abbildungen mit einer derart hohen Informationsfülle bereitgestellt, so dass eine zuverlässige und effiziente Charakterisierung von Hautläsionen, insbesondere bei einer Mehrzahl zu untersuchenden Hautbereichen, durch einen behandelnden Arzt nicht möglich ist.

Der vorliegenden Erfindung liegt die Aufgabe zugrunde die vorgenannten Nachteile des Standes der Technik zu überwinden oder wenigstens deutlich abzuschwächen. Insbesondere soll eine optimierte bildgebende Vorrichtung zur Erfassung und Charakterisierung einer Hautläsion bereitgestellt werden, welche eine effiziente und zuverlässige Erkennung von bösartigem und gutartigem Hautgewebe durch den behandelnden Arzt ermöglicht. Diese Aufgabe wird durch den Gegenstand der unabhängigen Ansprüche gelöst. Die Unteransprüche stellen vorteilhafte Weiterbildungen der vorliegenden Erfindung dar. Die Erfindung adressiert zudem weitere Probleme bzw. schlägt die Lösung zu weiteren Problemen vor, wie aus der nachfolgenden Beschreibung hervorgeht.

In einem ersten Aspekt betrifft die Erfindung eine Vorrichtung zur Erzeugung einer Mehrzahl von Abbildungen zur Unterstützung bei der Charakterisierung von Hautläsionen, aufweisend eine Beleuchtungseinheit zur selektiven Auflichtbeleuchtung eines Hautbereichs und Direkteinkopplung von Licht in einen Hautbereich, wobei die Beleuchtungseinheit eine Vielzahl von vorzugsweise gleichartigen LED Chips bzw. LED Lichtquellen aufweist, eine Messeinheit zur Auswertung einer Rückstreuung des von der Beleuchtungseinheit beleuchteten Hautbereichs, wobei die Messeinheit einen ersten Sensor zur Detektion von multispektralen oder hyperspektralen Bildinformationen und einen zweiten Sensor zur Detektion von RGB-Bildinformationen aufweist, und eine Verarbeitungs- und Ausgabeeinheit zur Abbildungserzeugung basierend auf den von der Messeinheit detektierten Bildinformationen, wobei die Beleuchtungseinheit einen ersten Hautbeleuchtungsbereich an einem Endabschnitt der Beleuchtungseinheit und einen davon beabstandeten zweiten Auflichtbeleuchtungsbereich aufweist, in welchem vorzugsweise jeweils eine Vielzahl von LED Chips angeordnet ist, und wobei die Steuereinheit ausgebildet ist, die Beleuchtungseinheit und die Messeinheit zur sequentiellen Detektion von Bildinformationen mittels Auflichtbeleuchtung und Direkteinkopplung von Licht anzusteuern.

Durch die vorliegende Erfindung wird die Erstellung von Abbildungen eines zu untersuchenden Hautbereichs und einer sich darin befindlichen Läsion nicht nur basierend auf detektierten multi- oder hyperspektralen Bildinformationen sondern auch basierend auf RGB (Rot,Grün,Bau) Bildinformationen eines erfassten, vom jeweiligen Hautbereich rückgestreuten, Lichts möglich. Durch die Bereitstellung von Abbildungen basieren auf den erfassten multi- oder hyperspektralen Bildinformationen sowie RGB Bildinformationen kann eine einfachere Analyse durch den behandelnden Arzt erfolgen. Insbesondere kann neben einer Detailansicht einer Vielzahl von Farbkanälen einer Abbildung basierend auf den detektierten multi- oder hyperspektralen Bildinformationen eine echtfarbige bzw. fotographische Abbildung des jeweiligen Hautbereichs basierend auf den detektierten RGB Bildinformationen dargestellt werden, wodurch insbesondere bei einer Vielzahl von zu analysierenden Hautbereichen eine einfachere Analyse und Zuordnung der erfassten Bildbereiche durch den behandelnden Arzt möglich ist. Zudem können die einzelnen RGB Kanäle zusätzlich zu vordefinierten Farbkanälen in einer Abbildung basierend auf den multi- oder hyperspektralen Bildinformationen zur Auswertung und Diagnose herangezogen werden.

Ebenso ermöglicht die vorliegende Erfindung durch die Beleuchtung mit vorzugsweise gleichartigen LED Chips eine wesentlich einfachere und zuverlässige Bauart des Messsystems im Vergleich zum Stand der Technik. Insbesondere muss hierbei im Gegensatz zum Stand der Technik keine aufwendige sequentielle Beleuchtung mittels Licht einer Vielzahl von unterschiedlichen Wellenlängen erfolgen.

In einer vorteilhaften Ausführungsform sind die LED Chips bzw. LED Lichtquellen so gewählt, dass diese in einem vorzugsweise kontinuierlichen Spektrum innerhalb des elektromagnetischen Spektrums emittieren. Das emittierte Spektrum umfasst vorzugsweise wenigstens den kompletten sichtbaren Bereich des elektromagnetischen Spektrums von ca. 380 bis 700nm. Das emittierte Spektrum umfasst vorzugsweise zudem wenigstens teilweise den ultravioletten Bereich des elektromagnetischen Spektrums von 10nm bis 380nm und/oder den Infrarotbereich von 700nm bis 1000nm. Die LED Chips sind vorzugswese derart ausgebildet, dass diese wenigstens Licht in denjenigen Wellenlängenbereichen emittieren, in welchen der erste und zweite Sensor der Messeinheit sensitiv ist. In einer besonders bevorzugten Ausführungsform sind die LED Chips derart ausgebildet, dass diese elektromagnetische Strahlung in einem Wellenlängenbereich von 400 bis 1000nm emittieren. Die LED Chips können hierbei derart ausgebildet sein, dass das emittierte Spektrum mehrere Peaks in unterschiedlichen Wellenlängenbereichen aufweist.

Die Beleuchtungseinheit weist vorzugsweise nur gleichartige LED Chips bzw. LED Lichtquellen auf. Die LED Chips können beispielsweise farbstoffkonvertierte LED Chips sein, welche das Primärlicht eines jeweiligen LED Chips durch einen ein- bzw. aufgebrachten Farbstoff wenigstens teilweise in Licht einer anderen Wellenlänge konvertieren, wobei sich ein resultierendes Mischlicht vorzugsweise weißes Licht ergibt. Alternativ können die LED Chips auch mehrere unterschiedliche LED Chips umfassen, welche vorzugsweise zusammen Licht im Bereich des UV Spektrums, des sichtbaren Spektrums und/oder des Infrarotspektrums emittieren. Beispielsweise können die LED Chips eine Mehrzahl von farbstoffkonvertierten Weißlicht LED Chips, UV LED Chips und Infrarot emittierenden LED Chips umfassen.

Die Beleuchtungseinheit ist ausgebildet, die Auflichtbeleuchtung und die Direkteinkopplung von Licht selektiv bereitzustellen. Hierbei steuert die Steuereinheit die Beleuchtungseinheit und die Messeinheit zur Erfassung der jeweiligen Bildinformationen bei der jeweiligen Beleuchtung an. Besonders bevorzugt ist die Steuereinheit ausgebildet, die Beleuchtungseinheit und Messeinheit zur sequentiellen Beleuchtung und Erfassung der jeweiligen Bildinformationen anzusteuern. Insbesondere erfolgt hierbei zunächst eine Auflichtbeleuchtung und anschließend eine Beleuchtung mittels Direkteinkopplung von Licht in den jeweiligen Hautbereich, oder umgekehrt. Die Reihenfolge kann beliebig gewählt werden und/oder auf eine eventuelle Ansteuerung des ersten und zweiten Sensors abgestimmt sein. Vorzugsweise erfolgt für den jeweiligen Hautbereich nur eine einzelne sequentielle Erfassung bzw. Messung bestehend aus einer Erfassung mittels Auflichtbeleuchtung und einer Erfassung mittels Direkteinkopplung.

Die Beleuchtungseinheit weist einen ersten Hautbeleuchtungsbereich auf, welcher an einem dem zu untersuchenden Hautbereich zugewandten Endabschnitt der Beleuchtungseinheit angeordnet ist. Zudem weist die Beleuchtungseinheit einen zweiten Autlichtbeleuchtungsbereich auf, welcher vom Hautbeleuchtungsbereich distanziert angeordnet ist. Der Hautbeleuchtungsbereich und der Auflichtbeleuchtungsbereich erstrecken sich vorzugsweise jeweils in einer ebenen Fläche und bilden daher jeweils eine Lichtquellenebene. Beide Bereiche weisen vorzugsweise eine Vielzahl von LED Chips auf. Der Hautbeleuchtungsbereich und/oder der Auflichtbeleuchtungsbereich sind vorzugsweise ringförmig oder rechteckförmig um einen Erfassungsbereich der Beleuchtungseinheit angeordnet ist.

Bei der Auflichtbeleuchtung werden vorzugsweise Bildinformationen aufgenommen bzw. detektiert, welche primär aus der Oberfläche des zu untersuchenden Hautbereichs und insbesondere der äußersten Hautschicht, der Stratum Corneum, stammen. Es können hierbei aber auch Bildinformationen aus den darunterliegenden Hautschichten, insbesondere der Stratum lucidum, der Stratum granulosum, der Stratum spinosum und der Stratum basale detektiert werden.

Bei der Direkteinkopplung von Licht werden vorzugsweise Bildinformationen aufgenommen bzw. detektiert, welche neben der äußersten Hautschicht, der Stratum Corneum, auch aus tieferliegenden Hautschichten stammen, d.h. wenigstens teilweise auch aus den darunter liegenden Schichten der Stratum lucidum, der Stratum granulosum, der Stratum spinosum und der Stratum basale. Bei der Direkteinkopplung von Licht kann ein an der Hautoberfläche reflektierter Anteil von Licht eliminiert werden. Hierdurch kann eine von der Auflichtbeleuchtung abweichende Bildinformation detektiert werden. Es kann insbesondere eine optimierte Transillumination des zu untersuchenden Hautbereichs erzielt werden.

Die vorzugsweise Bereitstellung einer Kombination von Auflichtbeleuchtung und Direkteinkopplung von Licht für den zu untersuchenden Hautbereich ermöglicht eine umfassende Erfassung und Detektion von spektralen Bildinformationen bei unterschiedlichen Transilluminationzuständen des zu untersuchenden Hautbereichs, wodurch eine optimierte Informationsausbeute zur Beurteilung des zu untersuchenden Hautbereichs bzw. einer sich darin befindlichen Läsion erzielt wird.

Die Auflichtbeleuchtung kann bei Benutzung der Vorrichtung außerhalb der Beleuchtung durch Direkteinkopplung, d.h. wenn die Direkteinkopplung nicht aktiviert ist, und/oder bei Bewegung der Vorrichtung aktiviert sein. Dies ermöglicht eine optimierte Platzierung der Beleuchtungseinheit auf dem zu untersuchenden Hautbereich.

Der erste Sensor der Messeinheit zur Detektion von multi- oder hyperspektralen Bildinformationen ist ein multi- oder hyperspektraler Sensor. Mit multi- oder hyperspektralen Bildinformationen werden vorliegend Intensitätsinformationen verstanden, welche in den jeweiligen Wellenlängenbereichen, für die der Sensor sensitiv ist, in der Rückstreuung des von der Beleuchtungseinheit beleuchteten Hautbereichs erfasst werden.

Der multispektrale Sensor ist vorzugsweise zur Detektion von elektromagnetischer Strahlung in wenigstens 4-12 vordefinierten Wellenlängenbereichen bzw. Bändern ausgebildet. Der hyperspektrale Sensor ist vorzugsweise zur Detektion von elektromagnetischer Strahlung in wenigstens 8-20 vordefinierten Wellenlängenbereichen bzw. Bändern ausgebildet.

Der multi- oder hyperspektrale Sensor ist vorzugsweise in einem Wellenlängenbereich von 400-1000nm sensitiv. Beispielsweise kann es sich bei dem multi- oder hyperspektralen Sensor um einen CMOS Sensor der Firma Silios handeln, welcher ein erfassbares Spektralband zwischen 400 und 1000nm und eine Auflösung von 1280×1024 Pixel aufweist.

Die vordefinierten einzelnen Wellenlängenbereiche bzw. Bänder, in denen der multi-/hyperspektrale Sensor sensitiv ist, umfassen vorzugsweise mehrere oder mehr bevorzugt alle der nachfolgenden Wellenlängen oder Wellenlängenbereiche, welche für die jeweils genannten nachstehenden Werte und Informationen zur Auswertung und Analyse des erfassten Hautbereichs herangezogen werden können:
420nm - Einbruch des Reflexionsspektrums von Haut
450nm - Oberflächliche Hautschicht
470nm - Oberflächliche Melanindetektion
500nm - Transmissionsspektrum
520nm-620nm - Absorption Oxyhämoglobin und Deoxyhämoglobin
660nm - Melanindetektion
760nm - Hämoglobin Absorptionsspitze
800nm - Absorption für Deoxyhämoglobin und Oxyhämoglobin
910nm - Peak für Oxyhämoglobin - Absorption
950nm - Peak für Wasser Absorption

Der zweite Sensor der Messeinheit zur Detektion von RGB Bildinformationen ist ein RGB Sensor. Mit RGB Bildinformationen werden vorliegend Intensitätsinformationen verstanden, welche in den jeweiligen rot, grünen und blauen Wellenlängenbereichen, für die der Sensor sensitiv ist, in der Rückstreuung des von der Beleuchtungseinheit beleuchteten Hautbereichs erfasst werden.

In einer bevorzugten Ausführungsform weist die Messeinheit einen Strahlenteiler auf. Dieser kann insbesondere ein Strahlenteilerwürfel sein. Der Strahlenteiler ist dazu ausgebildet, die erfasste Rückstreuung an den ersten und zweiten Sensor mit vorzugsweise gleicher Intensität bereitzustellen. Insbesondere trennt der Strahlenteiler die erfasste Rückstreuung in zwei Strahlengänge auf, welche auf den ersten und zweiten Sensor gerichtet werden.

Die Sensoren der Messeinheit sind vorzugsweise derart angeordnet oder ausgebildet, dass das jeweils erfasste Sichtfeld des Sensors übereinstimmt. Zur Einstellung des jeweiligen Sichtfelds bzw. Anpassung des Sichtfelds an den jeweiligen anderen Sensor kann die Messeinheit eine oder mehrere Optiken aufweisen. Insbesondere kann jedem der Sensoren eine Optik zur Fokussierung der von dem Strahlenteiler bereitgestellten Strahlengänge zugeordnet sein.

Die Steuereinheit zur Ansteuerung der Beleuchtungseinheit und der Messeinheit kann durch einen Rechner aufweisend beispielsweise einen Personal Computer (PC), Laptop oder Tablet und eine zugehörige Steuerungssoftware gebildet sei. Die Steuerungseinheit kann auch durch einen Mikroprozessor und/oder Mikrokontroller mit zugeordneten Speichermitteln zur Ausführung eines Steuerprogramms gebildet sein. Die Steuereinheit kann zudem eine Benutzerschnittstelle mit Eingabemitteln aufweisen.

Die Steuereinheit kann zudem eine Anpassung der multi- oder hyperspektralen und/oder RGB Darstellung bzw. Bildererzeugung mittels der Sensoren ermöglichen. Beispielsweise kann die Steuereinheit ausgebildet sein, die Belichtungsparameter wie Belichtungszeit, Blende. Gain und/oder Gamma einzustellen. Dies kann basierend auf vordefinierten Parametern, basierend auf gemessenen Parametern des zu untersuchenden Hautbereichs und/oder basierend auf einer Benutzereingabe erfolgen.

Die Verarbeitungs- und Ausgabeeinheit kann als Teil der Steuereinheit oder separat hierzu ausgebildet sein. Die Verarbeitungs- und Ausgabeeinheit umfasst vorzugsweise einen Softwarealgorithmus und/oder einen Prozessor zur Ausführung eines Softwarealgorithmus, mittels dem die Bildinformationen des ersten und zweiten Sensors in jeweilige Abbildungen umgewandelt werden. Zur Darstellung der Abbildungen umfasst die Ausgabeeinheit vorzugsweise ein Display oder einen Touchscreen. Zudem umfasst die Verarbeitungs- und Ausgabeeinheit zugeordnete Speichermittel zur Bereitstellung von Daten.

Die Verarbeitungs- und Ausgabeeinheit ist vorzugsweise ausgebildet, ein Multispektral- bzw. Hyperspektralbild des erfassten Hautbereichs mit einer Vielzahl von n-Farbkanälen darzustellen. Diese sind vorzugsweise mittels der Ausgabeeinheit selektiv durch einen Benutzer darstellbar. Die Verarbeitungs- und Ausgabeeinheit ist vorzugsweise auch ausgebildet, ein RGB Bild des erfassten Hautbereichs mit den drei Farbkanälen rot, grün und blau darzustellen. Auch diese Farbkanäle sind vorzugsweise mittels der Ausgabeeinheit selektiv durch einen Benutzer darstellbar. Besonders bevorzugt wird das RGB Bild als echtfarbige bzw. fotographische Wiedergabe des erfassten Hautbereichs dargestellt.

Die Verarbeitungs- und Ausgabeeinheit ist vorzugsweise ausgebildet, die erzeugten Abbildungen selektiv als jeweilige Kombination der RGB Bildinformationen mit den multi- oder hyperspektralen Bildinformationen unter Auflichtbeleuchtung oder Direkteinkopplung der Beleuchtung auszugeben. Auf diese Weise kann eine gleichzeitige Darstellung des jeweiligen Hautbereichs als vorzugsweise echtfarbiges RGB Bild und eine vorzugsweise selektiv änderbare Darstellung eines multi- oder hyperspektralen Bild erfolgen, auch jeweils selektiv für eine wählbare Beleuchtung. Das jeweilige RGB Bild und ein multi- oder hyperspektrales Bild können beispielsweise nebeneinander auf einem Display ausgegeben werden.

Die Verarbeitungs- und Ausgabeeinheit ist vorzugsweise ausgebildet eine wellenlängenabhängige Intensitätscharakteristik der erfassten Rückstreuung auszugeben, welche auf den detektierten Bildinformationen des ersten und/oder zweiten Sensors basiert. Die Intensitätscharakteristik wird vorzugsweise für den Wellenlängenbereich zwischen 400 bis 1000nm ausgegeben.

In einer bevorzugten Ausführungsform ist die Verarbeitungs- und Ausgabeeinheit dazu ausgebildet, eine Vorcharakterisierung und/oder Bewertung der von der Messeinheit erfassten Bildinformationen durchzuführen. Hierfür weist die Verarbeitungseinheit- und Ausgabeeinheit vorzugsweise einen Software-Algorithmus auf, welcher eine Vorcharakterisierung und/oder Bewertung der erfassten Bildinformationen vornehmen kann. Diese kann beispielsweise auf einer Charakterisierung und/oder Bewertung der erfassten Bildinformationen hinsichtlich vordefinierter Kriterien oder unter Abgleich mit Vergleichsinformationen, beispielsweise aus einer Datenbank, durchgeführt werden. Beispielsweise kann eine Vorcharakterisierung und/oder Bewertung unter Anwendung bzw. Analyse hinsichtlich der bekannten "ABCDE"-Regeln erfolgen, d.h. hinsichtlich einer Asymmetrie, Begrenzung. Färbung, Durchmesser und Veränderung analysiert werden. Bei der Vorcharakterisierung und/oder Bewertung kann auch eine Analyse basierend auf Vergleichsdaten bzw. Vergleichsbildinformationen erfolgen, welche mittels einer histologischen Untersuchung verifiziert wurden. Basierend auf der oben genannten Vorcharakterisierung kann eine Einstufung bzw. Bewertung hinsichtlich Relevanz bzw. Gefährlichkeit einer Hautläsion vorgenommen werden.

Die Verarbeitungs- und Ausgabeeinheit umfasst vorzugsweise einen Algorithmus, welcher eine Analyse der von der Messeinheit erfassten Bildinformationen mittels künstlicher Intelligenz (KI), vorzugsweise mittels eines Deep-Learning Algorithmus, durchführt. Der KI-Algorithmus kann die oben beschriebene Charakterisierung und/oder Bewertung durchführen oder diese unterstützen. Der KI-Algorithmus kann hierbei auf die multi-/hyperspektralen Bildinformationen und/oder auf die RGB Bildinformationen gestützt sein. Der KI-Algorithmus kann mittels Vergleichsdaten trainiert werden, welche beispielsweise auf Bildinformationen und durch histologische Untersuchung verifizierte Ergebnisse bzw. Diagnosen basieren.

Die Verarbeitungs- und Ausgabeeinheit kann ausgebildet sein, ein Ergebnis der Vorcharakterisierung und/oder Bewertung des Hautbereichs mittels einer Bewertungsangabe beispielsweise in Skalenform und/oder in Form eines numerischen Werts auszugeben. Die Darstellung dieser Ausgabe kann gleichzeitig mit der Abbildungsdarstellung basierend auf den multi- oder hyperspektralen Bildinformationen und RGB-Bildinformationen erfolgen.

Die Verarbeitungs- und Ausgabeeinheit kann ausgebildet sein, bei einer Mehrzahl von mittels der Vorrichtung untersuchten bzw. erfassten Hautbereichen diejenigen Abbildungen oder Abbildungsserien für spezifische Hautbereiche graphisch hervorzuheben, welche durch die Charakterisierung und/oder Bewertung als von höherer Relevanz eingestuft wurden. Diese können dann einem behandelnden Arzt zur näheren Analyse dargestellt werden und/oder unter der Mehrzahl der erfassten Hautbereichen zur Analyse besonders hervorgehoben oder markiert werden.

In einer bevorzugten Ausführungsform weist die Vorrichtung ein mobiles bzw. tragbares Handgerät und eine mit diesem verbundene Recheneinheit auf. Das Handgerät umfasst vorzugsweise die Beleuchtungseinheit und die Messeinheit. Diese sind vorzugsweise in einem gemeinsamen Gehäuse untergebracht. Die Recheneinheit kann vorzugsweise ein Rechner. Laptop oder Tablet sein, welcher vorzugsweise die Verarbeitungs- und Ausgabeeinheit sowie die Steuereinheit umfasst. Die Recheneinheit umfasst zudem vorzugsweise ein Display und/oder eine Benutzerschnittstelle.

In einem weiteren Aspekt betrifft die Erfindung ein Verfahren zur Erzeugung einer Mehrzahl von Abbildungen zur Unterstützung bei der Charakterisierung von Hautläsionen, vorzugsweise unter Verwendung der oben beschriebenen Vorrichtung, aufweisend die Schritte:
- Beleuchtung eines zu untersuchenden Hautbereichs mittels einer Auflichtbeleuchtung und Direkteinkopplung von Licht in den Hautbereich durch eine Vielzahl von vorzugsweise gleichartigen LED Chips bzw. LED Lichtquellen,
- Erfassung einer Rückstreuung des zu untersuchenden Hautbereichs für die jeweilige Beleuchtung,
- Detektion von multi- oder hyperspektralen Bildinformationen in der erfassten Rückstreuung mittels eines ersten Sensors,
- Detektion von RGB Bildinformationen in der erfassten Rückstreuung mittels eines zweiten Sensors, und
- Verarbeitung der detektierten Bildinformationen zur Erzeugung und Ausgabe von Abbildungen.

Die Auflichtbeleuchtung und die Direkteinkopplung von Licht erfolgen mittels zwei in unterschiedlicher Distanz zum zu untersuchenden Hautbereich angeordneten Lichtquellenebenen. Die Lichtquellenebenen weisen jeweils eine Vielzahl von vorzugsweise hinsichtlich des emittierten Spektrums gleichartigen LED Chips auf. Die Auflichtbeleuchtung und die Direkteinkopplung von Licht erfolgen vorzugsweise sequentiell hintereinander. Hierbei erfolgen vorzugsweise für den zu untersuchenden Hautbereich jeweils nur eine einzelne Auflichtbeleuchtung und eine einzelne Direkteinkopplung von Licht.

In einem bevorzugten Ausführungsbeispiel erfolgen die Detektion der multi- oder hyperspektralen Bildinformationen und die Detektion von RGB Bildinformationen gleichzeitig. Dies kann mit einer Erfassung der rückgestreuten Lichtstrahlen jeweils bei der Auflichtbeleuchtung und bei der Direkteinkopplung von Licht erfolgen. Die erfassten rückgestreuten Lichtstrahlen können mittels eines Strahlenteilers dem ersten und zweiten Sensor zugeführt werden, welcher das erfasste Licht in zwei Strahlengänge aufteilt. Der erste und zweite Sensor sind vorzugsweise derart ausgebildet oder angeordnet, dass diese den gleichen Sichtbereich bzw. das gleiche Sichtfeld aufweisen bzw. abdecken.

Die Verarbeitung der Bildinformationen erfolgt vorzugsweise derart, dass aus den multioder hyperspektralen Bildinformationen eine multi- oder hyperspektrale Abbildung mit einer Vielzahl selektiv darstellbarer Farbkanäle erzeugt wird, jeweils für die Beleuchtung mittels Auflichtbeleuchtung und mittels Direkteinkopplung der Beleuchtung. Aus den RGB Bildinformationen wird vorzugsweise eine RGB Abbildung mit drei selektiv einzeln oder kombiniert darstellbaren Farbkanälen erzeugt, jeweils für die Beleuchtung mittels Auflichtbeleuchtung und mittels Direkteinkopplung. Zusätzlich kann aus den multi- oder hyperspektralen Bildinformationen und/oder aus den RGB Bildinformationen eine Abbildung einer wellenlängenabhängigen Intensitätscharakteristik in vordefinierbaren Spektralbereichen, vorzugsweise innerhalb eines Bereichs von 400nm bis 1000nm, erzeugt werden.

Die bei der Verarbeitung erzeugten Abbildungen können dann einem Benutzer bzw. behandelnden Arzt zur weiteren Analyse oder Diagnose dargestellt bzw. ausgegeben werden. Eine Ausgabe der erzeugten Abbildungen erfolgt vorzugsweise als Kombination von erzeugten Abbildungen basierend auf RGB Bildinformationen und multi- oder hyperspektralen Bildinformation unter Auflichtbeleuchtung, als Kombination von erzeugten Abbildungen basierend auf RGB Bildinformationen und multi- oder hyperspektralen Bildinformationen unter Direkteinkopplung der Beleuchtung, und/oder als Abbildung einer wellenlängenabhängige Intensitätscharakteristik in vordefinierten Spektral bereichen, vorzugsweise zwischen 400nm bis 1000nm. Die Ausgabe der Abbildungen ist hierbei vorzugsweise selektiv wählbar.

In einer bevorzugten Ausführungsform weist das erfindungsgemäße Verfahren den weiteren Schritt einer Vorcharakterisierung und/oder Bewertung der von der Messeinheit erfassten Bildinformationen auf. Dieser erfolgt vorzugsweise vor der Ausgabe der erzeugten Abbildungen. Die Vorcharakterisierung und/oder Bewertung kann basierend auf einem Algorithmus mit künstlicher Intelligenz erfolgen, vorzugsweise mit einem Deep-Learning Algorithmus.

Zur Vermeidung von Wiederholungen wird auf die oben beschriebenen Merkmale der erfindungsgemäßen Vorrichtung verwiesen, welche gleichermaßen als für das erfindungsgemäße Verfahren offenbart gelten sollen und umgekehrt.

Weitere vorteilhafte Merkmale, Vorteile und Einzelheiten der Erfindung ergeben sich aus der nachfolgenden Beschreibung bevorzugter Ausführungsbeispiele sowie anhand der Figuren. Diese zeigen in:
**Fig. 1** eine schematische Ansicht einer bevorzugten Ausführungsform der erfindungsgemäßen Vorrichtung,
**Fig. 2** eine schematische Ansicht der Auflichtbeleuchtung und der Direkteinkopplung von Licht mittels einer bevorzugten Ausführungsform der erfindungsgemäßen Beleuchtungseinheit und die jeweilige Erfassung einer Rückstreuung,
**Fig. 3a** eine perspektivische Schnittansicht einer bevorzugten Ausführungsform der Beleuchtungseinheit der erfindungsgemäßen Vorrichtung,
**Fig. 3b** eine perspektivische Ansicht einer bevorzugten Ausführungsform eines Handgeräts aufweisend die Beleuchtungseinheit und Messeinheit der erfindungsgemäßen Vorrichtung,
**Fig. 4** eine schematische Ansicht der Verfahrensschritte einer bevorzugten Ausführungsform des erfindungsgemäßen Verfahrens, und
**Fig. 5a, 5b** schematische Ansichten von erzeugten Abbildungen basierend auf den detektierten Bildinformationen.

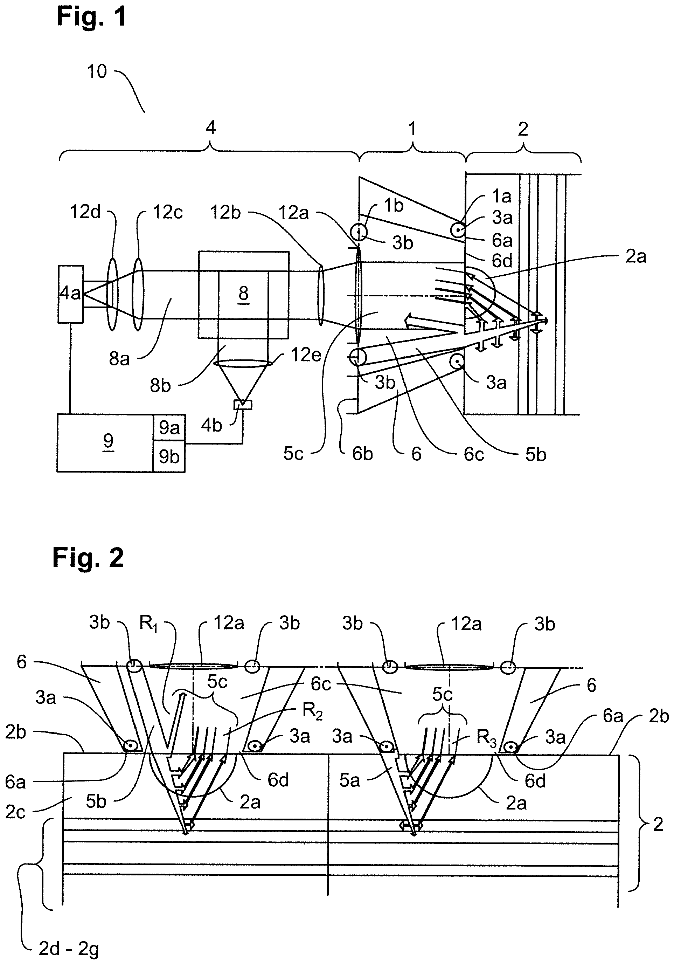

**Fig. 1** zeigt schematisch eine bevorzugte Ausführungsform der erfindungsgemäßen Vorrichtung 10. Diese weist eine Beleuchtungseinheit 1 zur Beleuchtung eines Hautbereichs 2, eine Messeinheit 4 und eine Steuereinheit 9 zur Ansteuerung der Beleuchtungseinheit 1 und der Messeinheit 4 auf.

Die Beleuchtungseinheit 1 weist einen vorzugsweise kegelstumpfförmigen Erfassungskörper 6 mit einem dem Hautbereich 2 zugewandten distalen Endabschnitt 6a und einem diesem gegenüberliegenden proximalen Abschnitt 6b auf. Der Erfassungskörper 6 weist einen inneren, hohlen Erfassungsbereich 6c auf, welcher zum Endabschnitt 6a mittels einer vorzugsweise zentral angeordneten Öffnung 6d geöffnet ist. Der Erfassungsbereich 6c ist vorzugsweise auf den Endabschnitt 6a bzw. die Öffnung 6d verjüngend bzw. sich auf den gegenüberliegenden Abschnitt 6b erweiternd ausgebildet. Der Erfassungsbereich 6c kann alternativ auch einen im Wesentlichen homogenen Durchmesser aufweisen.

Die Beleuchtungseinheit 1 weist eine Vielzahl von LED Chips bzw. LED Lichtquellen 3a,3b auf. Diese sind in einem ersten Hautbeleuchtungsbereich 1a an dem Endabschnitt 6a der Beleuchtungseinheit 1 und in einem zweiten Auflichtbeleuchtungsbereich 1b, welcher vom ersten Hautbeleuchtungsbereich 1a beabstandet ist, angeordnet. Der zweite Auflichtbeleuchtungsbereich 1b ist insbesondere zurückversetzt im Erfassungskörper 6 angeordnet. Mittels der LED Chips 3a im Hautbeleuchtungsbereich 1a kann eine Direkteinkopplung 5a (vgl. auch Fig. 2) von Licht in den Hautbereich 2 und in eine sich darin befindliche Läsion 2a erfolgen. Mittels der LED Chips 3b im Auflichtbeleuchtungsbereich 1b kann eine Auflichtbeleuchtung 5b des Hautbereichs 2 und der darin befindlichen Läsion 2b erfolgen.

Die Beleuchtungseinheit 1 kann zudem optische Mittel und/oder Einstellmittel aufweisen, welche beispielsweise zur Einstellung und/oder Fokussierung des von den LED Chips emittierten Lichts dienen und/oder dazu ausgebildet sind, eine Rückstreuung des von der Beleuchtungseinheit beleuchteten Hautbereichs zu erfassen und/oder zur Messeinheit zu lenken.

Die LED Chips 3a,3b sind vorzugsweise gleichartige LED Chips bzw. LED Chips, welche in einem im Wesentlichen gleichen Spektrum emittieren. Die LED Chips 3a,3b emittieren vorzugsweise ein kontinuierliches, d.h. zusammenhängendes Spektrum, welches vorzugsweise zwischen 400 und 1000nm liegt.

Die Messeinheit 4 der Vorrichtung ist vorzugsweise ein optisches System und ausgebildet eine Rückstreuung 5c des beleuchteten Hautbereichs 2 bzw. der sich darin befindlichen Läsion 2a auszuwerten. Bei der Rückstreuung 5c handelt es sich von dem Hautbereich 2 unter der jeweiligen Beleuchtung 5a,5b zurückgestreuten Lichtstrahlen. Die Messeinheit 4 weist einen ersten Sensor 4a zur Detektion von multi- oder hyperspektralen Bildinformationen und einen zweiten Sensor 4b zur Detektion von RGB-Bildinformationen auf. Der erste Sensor 4a kann ein multispektraler Sensor zur Detektion von elektromagnetischer Strahlung in wenigstens 4-11 vordefinierten Wellenlängenbereichen bzw. Bändern oder ein hyperspektraler Sensor zur Detektion von elektromagnetischer Strahlung in wenigstens 12- bis 20 vordefinierten Wellenlängenbereichen bzw. Bändern sein.

Die Messeinheit 4 weist vorzugsweise einen Strahlenteiler 8 auf, welcher die erfassten rückgestreuten Lichtstrahlen bzw. die Rückstreuung 5c an den ersten und zweiten Sensor 4a,4b bereitstellt. Der Stahlenteiler 8 ist vorzugsweise ein Strahlenteilerwürfel, welcher die Rückstreuung 5c in zwei Strahlengänge 8a,8b mit vorzugsweise gleicher Intensität aufteilt und den Sensoren 4a,4b zuführt.

Die Messeinheit 4 kann zudem Optiken 12a-12d aufweisen, welche die Rückstreuung 5c und/oder die Strahlengänge 8a,8b bündeln bzw. fokussieren. Die Optiken 12a-12d sind vorzugsweise derart ausgebildet bzw. angeordnet, dass das erfasste Sichtfeld für beide Sensoren übereinstimmt. Die Messeinheit 4 kann zudem Einstellmittel (nicht gezeigt) zur Anpassung bzw. Justierung der Optiken 12a-12d aufweisen.

Die Steuereinheit 9 der Vorrichtung 10 ist zur Ansteuerung der Beleuchtungseinheit 1 und der Messeinheit 4 ausgebildet. Insbesondere koordiniert die Steuereinheit 9 die selektive Aktivierung der Beleuchtungseinheit 1 und der Messeinheit 4 derart, dass die oben genannten Bildinformationen bei der jeweiligen Beleuchtung erfasst und ausgewertet werden können. Die Steuereinheit 9 ist ausgebildet, die Beleuchtungseinheit 1 zur selektiven Auflichtbeleuchtung 5b und Direkteinkopplung 5a in den Hautbereich 2 anzusteuern. Vorzugsweise steuert die Steuereinheit 9 die Beleuchtungseinheit 1 und die Messeinheit 4 derart an, dass die Auflichtbeleuchtung 5b und Direkteinkopplung 5a für die Analyse des jeweiligen Hautbereichs 2 sowie die jeweilige Erfassung und Auswertung der Bildinformationen sequentiell durchgeführt wird.

Die Vorrichtung 10 weist zudem eine Verarbeitungs- und Ausgabeeinheit 9a zur Abbildungserzeugung basierend auf den von der Messeinheit 4 detektierten Bildinformationen auf. Diese kann wie dargestellt Teil der Steuereinheit 9 sein oder alternativ separat hierzu ausgebildet sein. Die Verarbeitungs- und Ausgabeeinheit 9a kann einen Softwarealgorithmus und/oder einen Prozessor zur Ausführung eines Softwarealgorithmus aufweisen, mittels dem die Bildinformationen des ersten und zweiten Sensors 4a,4b in jeweilige Abbildungen umgewandelt werden. Zur Darstellung der Abbildungen umfasst die Ausgabeeinheit vorzugsweise ein Display oder einen Touchscreen 9b. Zudem umfasst die Verarbeitungs- und Ausgabeeinheit zugeordnete Speichermittel (nicht gezeigt) zur Bereitstellung von Daten und/oder eine Schnittstelle zur Anbindung an einen externeren Datenspeicher und/oder eine Datenbank (nicht gezeigt).

**Fig. 2** zeigt eine schematische Ansicht der Auflichtbeleuchtung 5b und der Direkteinkopplung 5a von Licht mittels der Beleuchtungseinheit 1 in den zu untersuchenden Hautbereich 2 aufweisend eine Läsion 2a.

Für die Untersuchung des jeweiligen Hautbereichs 2 bzw. der Läsion 2a wird die Beleuchtungseinheit 1 mit der Öffnung 6d im distalen Endabschnitt 6a des Erfassungskörpers 6 auf den jeweiligen Hautbereich 2 aufgesetzt, derart, dass der Erfassungsbereich 6c die Hautläsion umgibt. Zwischen dem Endabschnitt 6a und der Oberfläche der Haut 2b kann ein vor dem Aufsetzen der Beleuchtungseinheit 1 auf den Hautbereich 2 aufgetragenes Immersionsöl zur Anpassung des Brechungsindexes vorhanden sein.

Bei der links in der Fig. 2 abgebildeten Auflichtbeleuchtung 5b werden die LED Chips 3b, welche distanziert zur Hautoberfläche 2b angeordnet sind aktiviert, wodurch die Auflichtbeleuchtung 5b bereitgestellt wird. In der gezeigten Figur ist zur Vereinfachung lediglich ein Strahlengang 5b einer einzelnen LED 3b dargestellt. Hierbei trifft das von den LED Chips 3b emittierte Licht 5b mit einem vordefinierten Spektrum auf den Hautbereich 2 und wird teilweise von der Hautoberfläche 2b reflektiert (siehe Reflexionsanteil R1 in Fig. 2). Der nicht reflektierte Teil des Lichts 5b wird in die unteren Hautschichten, wie der Stratum corneum 2c, und teilweise auch den darunter liegenden Schichten Stratum lucidum 2d, Stratum granulosum 2e, Stratum spinosum 2f, Stratum basale 2g eingekoppelt. Hierbei erfolgt insbesondere eine Transillumination des Hautbereichs 2 und der Läsion 2a.

Die aus der Transillumination hervorgehende Rückstrahlung bzw. Reflexion bildet den Reflexionsanteil R2, welcher von der Beleuchtungseinheit 2 bzw. dem Erfassungsbereich 6c erfasst wird. Die Reflexionsanteile R1 und R2 bilden die diffuse Rückstreuung 5c des Hautbereichs 2 durch die Auflichtbeleuchtung 5b. Diese wird vom Erfassungskörper 6 und insbesondere von einer darin angeordneten Optik 12a erfasst und kann dann an die Messeinheit 4 zur Auswertung bzw. Analyse weitergeleitet werden.

Bei der rechts in der Fig. 2 abgebildeten Direkteinkopplung 5a werden die LED Chips 3a, welche im Haubeleuchtungsbereich 1a am distalen Endabschnitt 6a und somit vorzugsweise direkt an bzw. in der Auflagefläche zur Hautoberfläche 2b angeordnet sind aktiviert, wodurch die Direkteinkopplung 5a bereitgestellt wird. In der gezeigten Figur ist zur Vereinfachung lediglich ein Strahlengang 5a einer einzelnen LED 3a dargestellt. Hierbei trifft das von den LED Chips 3a emittierte Licht 5a mit einem vordefinierten Spektrum auf den Hautbereich 2, wobei durch die vorzugsweise direkte Auflage der LED Chips 3a auf der Hautoberfläche 2b eine Reflexion an der Oberfläche 2b eliminiert wird.

Mittels des eingekoppelten Lichts 5a erfolgt eine Transillumination des Hautbereichs 2 und der Läsion 2a. Insbesondere erfolgt hierbei eine Direkteinkopplung des Lichts in die Stratum corneum 2c, sowie vorzugsweise auch wenigstens teilweise in die darunter liegenden Schichten Stratum lucidum 2d, Stratum granulosum 2e, Stratum spinosum 2f und/oder Stratum basale 2g. Die hieraus hervorgehende Rückstrahlung bzw. Reflexion bildet einen Reflexionsanteil R3, welcher von der Beleuchtungseinheit 2 bzw. dem Erfassungsbereich 6c erfasst wird. Dieser stellt die diffuse Rückstreuung 5c des Hautbereichs 2 durch die Direkteinkopplung 5a dar, welche vom Erfassungskörper 6 und insbesondere von einer darin angeordneten Optik 12a erfasst und dann an die Messeinheit 4 zur Auswertung bzw. Analyse weitergeleitet werden kann.

Bei den zwei Beleuchtungsmodi, der Auflichtbeleuchtung 5b und der Direkteinkopplung 5a werden jeweils sowohl multi-/hyperspektrale Bildinformationen als auch RGB Bildinformationen aus der diffusen Rückstreuung 5c detektiert. Die zwei Beleuchtungsmodi werden vorzugsweise sequentiell durchgeführt. Dies bedeutet, dass der jeweilige zu untersuchende Hautbereich 2 bzw. die jeweilige Läsion 2a jeweils einmal mittels Auflichtbeleuchtung 5b und einmal mittels Direkteinkopplung 5a beleuchtet wird, wobei die jeweiligen Bildinformationen detektiert und anschließend ausgewertet werden können. Die notwendige Beleuchtungszeit liegt bei jedem der Beleuchtungsmodi bei vorzugsweise circa 1 bis 15 Sekunden, mehr bevorzugt zwischen 1-10 Sekunden, und am bevorzugtesten zwischen 1-5 Sekunden. Die Auflichtbeleuchtung kann bei Benutzung der Vorrichtung außerhalb der Beleuchtung durch Direkteinkopplung, d.h. wenn die Direkteinkopplung nicht aktiviert ist, und/oder bei Bewegung der Vorrichtung bzw. der Beleuchtungseinheit aktiviert sein. Hierbei kann die Auflichtbeleuchtung insbesondere dauerhaft außerhalb der Aktivierung der Direkteinkopplung aktiviert sein.

Die Verarbeitungs- und Ausgabeeinheit 9a erstellt anschließend Abbildungen zur Unterstützung eines behandelnden Arztes bei der Charakterisierung der Hautläsion 2a basierend auf den von den Sensoren 4a,4b der Messeinheit 4 detektierten Bildinformationen. Diese können dann mittels des Displays oder Touchscreens 9b dargestellt werden. Hierbei kann beispielsweise eine selektive Anzeige der erzeugten Abbildungen nach jeweiligem Beleuchtungsmodus 5a,5b oder nach gewünschtem Farbkanal der detektierten Bildinformationen erfolgen.

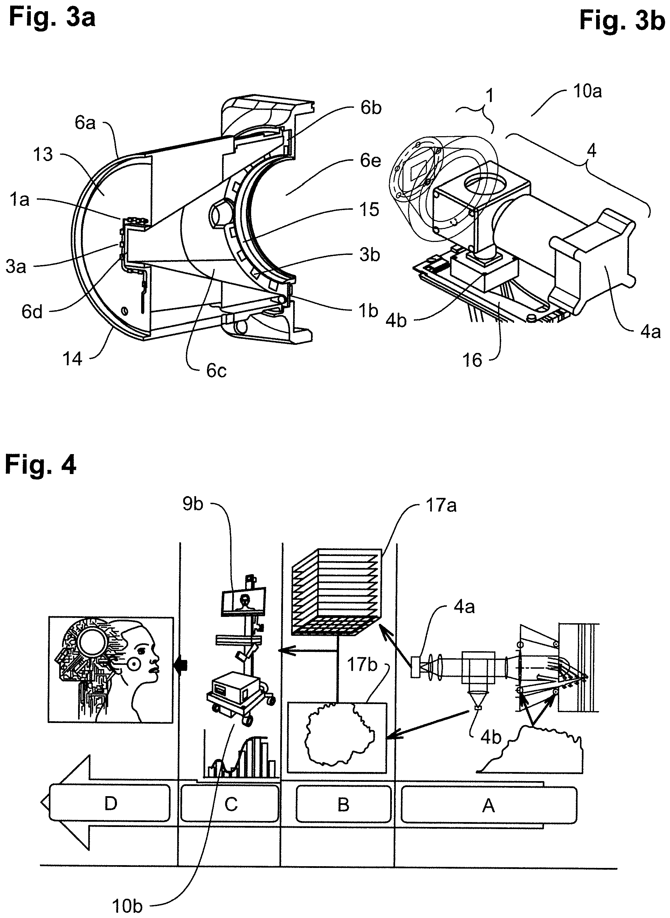

**Fig. 3a** zeigt eine perspektivische Schnittansicht einer bevorzugten Aus-führungsform der Beleuchtungseinheit 1 der erfindungsgemäßen Vorrichtung 10.

Der Erfassungskörper 6 hat hierbei eine zylindrische Außenform. Der Körper 6 kann jedoch auch eine beliebige andere Form aufweisen. Der im Erfassungskörper 6 ausgebildete Erfassungsbereich 6c weist eine kegelstumpfförmige Ausgestaltung auf, die sich vom distalen Endabschnitt 6a bzw. von der Öffnung 6d hin zum gegenüberliegenden proximalen Ende 6b erweitert. Eine Innenumfangsfläche des Erfassungsbereichs 6c kann reflektierend ausgebildet sein. Der distale Endabschnitt 6a weist einen darin geformten Rücksprung 13 auf, welcher den Hautbeleuchtungsbereich 1a aufweist, der die Öffnung 6d umgibt. Die LED Chips 3a des Hautbeleuchtungsbereichs 1a sind hierbei vorzugsweise im gleichmäßigen Abstand zueinander um die Öffnung 6d angeordnet. Die LED Chips 3a können hierbei im Wesentlichen rechteckförmig um die Öffnung 6d, oder ring- bzw. kreisförmig um die Öffnung 6d angeordnet sein. Die LED Chips 3a sind vorzugsweise derart angeordnet, dass diese beim Kontakt des distalen Endabschnitts 6a oder einer vorderen Kontaktfläche 14 davon direkt auf der Hautoberfläche 2b aufliegen. Die LED Chips 3a sind hierbei vorzugsweise in einer Ebene angeordnet. Bei einer ringförmigen Anordnung sind die LED Chips 1a vorzugsweise in homogenem radialen Abstand zu einer zentralen Mittelachse des Erfassungskörpers 6 bzw. der Öffnung 6d angeordnet.

Der Erfassungskörper 6 weist im proximalen Abschnitt 6b einen Rücksprung oder Absatz 15 auf. Dieser bildet den Auflichtbeleuchtungsbereich 1b mit den LED Chips 3b. Die LED Chips 1b sind ring- bzw. kreisförmig um eine rückwärtige Öffnung 6e des Erfassungskörpers angeordnet. Die Öffnung 6e ist dazu ausgebildet eine Optik 12a (siehe Fig. 1) aufzunehmen. Die LED Chips 3b des Auflichtbeleuchtungsbereichs 1b sind vorzugsweise in homogenen Abstand zueinander und vorzugsweise in konstantem radialen Abstand zu einer zentralen Mittelachse des Erfassungskörpers 6 bzw. der Öffnungen 6d und 6e angeordnet.

**Fig. 3b** zeigt eine perspektivische Ansicht einer bevorzugten Ausführungsform eines mobilen bzw. tragbaren Handgeräts 10a der Vorrichtung 10. Das Handgerät 10a umfasst die Beleuchtungseinheit 1 und die Messeinheit 4 in einem gemeinsamen Gehäuse. Das Handgerät 10a kann einen Griff oder eine Halterung 16 aufweisen.

Das Handgerät 10a ist vorzugsweise mit einer Recheneinheit 10b (siehe Fig. 4) der Vorrichtung 10 verbunden. Die Recheneinheit 10b kann vorzugsweise ein Rechner, Laptop oder Tablet sein, welcher die Verarbeitungs- und Ausgabeeinheit 9a sowie die Steuereinheit 9 umfasst. Die Recheneinheit umfasst zudem die Display und/oder Benutzerschnittstelle 9b.

**Fig. 4** zeigt eine schematische Ansicht der Verfahrensschritte einer bevorzugten Ausführungsform des erfindungsgemäßen Verfahrens.

In einem ersten Schritt A erfolgt die Beleuchtung eines zu untersuchenden Hautbereichs 2 mittels der Auflichtbeleuchtung 5b und der Direkteinkopplung 5a von Licht in den Hautbereich 2 wie oben beschrieben. Das jeweils zurückgestreute und/oder reflektierte Licht 5c wird von der Beleuchtungseinheit 1 erfasst und mittels der Messeinheit 4 ausgewertet. Insbesondere erfolgt hierbei eine Detektion von multispektralen oder hyperspektralen Bildinformationen in der Rückstreuung 5c mittels des ersten Sensors 4a und die eine Detektion von RGB Bildinformationen in der Rückstreuung 5c mittels des zweiten Sensors 4b.

In einem zweiten Schritt B erfolgt die Verarbeitung der detektierten Bildinformationen zu Abbildungen. Insbesondere wird basierend auf den multispektralen oder hyperspektralen Bildinformationen des ersten Sensors 4a, für jeden Beleuchtungsmodus wenigstens eine multi- bzw. hyperspektrale Abbildung 17a erstellt, welche eine Vielzahl von n-Farbkanälen aufweist. Basierend auf den RGB Bildinformationen des zweiten Sensors 4b wird für jeden Beleuchtungsmodus wenigstens eine RGB Abbildung 17b erstellt, welche drei Farbkanäle aufweist.

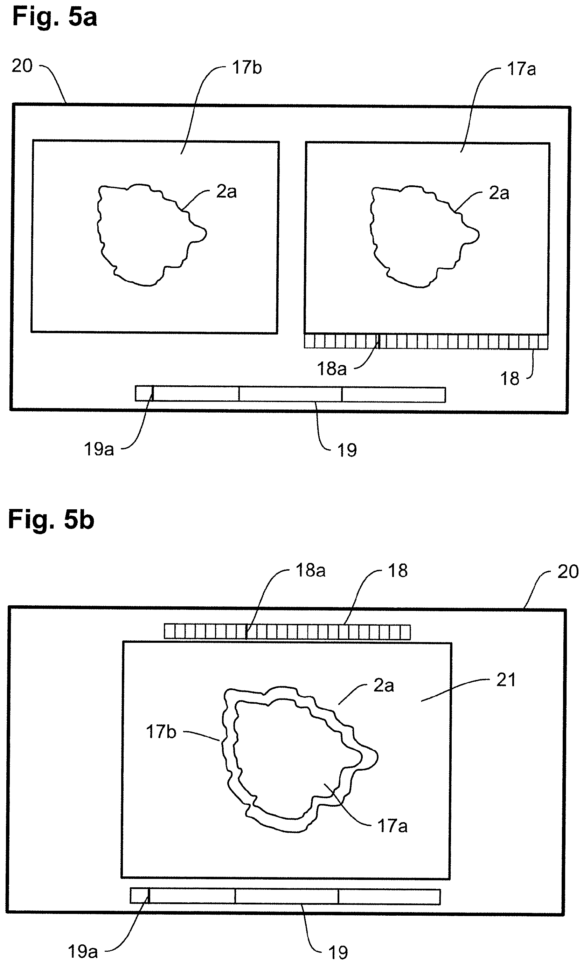

In einem dritten Schritt C erfolgt die Ausgabe der erzeugten Abbildungen 17a,17b an dem Rechner 10b. Dies wird untenstehend mit Verweis auf die Fig. 5a,5b näher beschrieben.

In einem vierten optionalen Schritt D erfolgt eine Vorcharakterisierung und/oder Bewertung der von der Messeinheit 4 erfassten Bildinformationen. Hierbei werden die erfassten Bildinformationen und/oder die basierend auf diesen Informationen erzeugten Abbildungen 17a,17b hinsichtlich vordefinierter Kriterien, wie den bekannten "ABCDE"-Regeln, und/oder unter Abgleich mit Vergleichsinformationen, beispielsweise aus einer Datenbank, analysiert. Basierend hierauf kann eine Einstufung bzw. Bewertung hinsichtlich Relevanz bzw. Gefährlichkeit einer Hautläsion auf dem Rechner 10b bzw. an einen Benutzer oder behandelnden Arzt ausgegeben werden. Schritt D kann eine Auswertung der detektierten Bildinformationen und/oder der basierend auf diesen Informationen erzeugten Abbildungen 17a,17b mittels eines künstlichen Intelligenz (KI) Algorithmus umfassen. Dieser kann die oben beschriebene Charakterisierung und/oder Bewertung durchführen oder diese unterstützen.

**Fig. 5a, 5b** zeigen schematische Ansichten von erzeugten Abbildungen basierend auf den detektierten Bildinformationen. Die Abbildungen werden mittels der Ausgabeeinheit 9b wie beispielsweise einem Display des Rechners 10b ausgegeben. Die Fig. 5a,5b zeigen hierbei zwei bevorzugte Bildschirmdarstellungen 20 des Displays.

In **Fig. 5a** ist eine erste bevorzugte Bildschirmdarstellungen 20 gezeigt, wobei neben einem Multi- oder Hyperspektralbild 17a das zugehörige RGB Bild 17b einer Hautläsion 2a dargestellt wird. Der Bildschirmausschnitt bzw. die Bildgröße des Hautbereichs 2 aufweisend die Läsion 2a ist für beide Bilder 17a,17b vorzugsweise gleich. Dies wird vorzugsweise durch das im Wesentlichen gleiche Sichtfeld des ersten und zweiten Sensors 4a,4b ermöglicht.

Das Hyperspektralbild 17a und das RGB Bild 17b sind vorzugsweise im gleichen Belichtungsmodus aufgenommen, d.h. unter Auflichtbeleuchtung oder Direkteinkopplung. Bei dem RGB Bild 17b handelt es sich vorzugsweise um eine fotographische Darstellung des erfassten Hautbereichs 2, während das Hyperspektralbild 17a eine Darstellung mit einer Vielzahl von Farbkanälen aufweist. Diese sind vorzugsweise selektiv im Bild 17a darstellbar und beispielsweise mittels zugehöriger Einstellmittel 19 auswählbar. Die Einstellmittel 19 können ein Einstellfeld sein, welches mittels einer Benutzerschnittstelle wie beispielsweise einer Maus oder Tastatur anwählbar und/oder veränderbar ist. Das Einstellfeld kann die Auswahl oder Angabe einer bestimmten Wellenlänge 18a oder bestimmten Strukturen wie beispielsweise bestimmten Spektren für Hämoglobin, Desoxyhämoglobin, Melanin etc. ermöglichen, welche dann im Hyperspektralbild 17a angezeigt werden.

Neben den oben genannten Abbildungen 17a,17b wird vorzugsweise ein Vorcharakterisierungs- oder Bewertungswert 19a ausgegeben bzw. abgebildet. Dies kann ein numerischer Wert sein oder eine Darstellung auf einer beispielsweise farblichen Skala 19. Der Vorcharakterisierungs- oder Bewertungswert 19a wird vorzugsweise im oben beschriebenen Schritt D (siehe **Fig. 4****)** ermittelt und dient einem behandelnden Arzt unterstützend zur besseren Analyse und Charakterisierung der Läsion 2a.

In der Bildschirmdarstellung 20 kann zusätzlich eine wellenlängenabhängige Intensitätscharakteristik für einen oder mehrere vordefinierte Spektralbereiche dargestellt werden (nicht gezeigt).

In **Fig. 5b** ist eine zweite bevorzugte Bildschirmdarstellungen 20 gezeigt, wobei das RGB Bild 17b und das zugehörige Hyperspektralbild 17a in einer überlagerten Abbildung 21 dargestellt werden. Das jeweilige überlagerte Bild 21 kann für eine gewünschte Beleuchtung, d.h. Auflichtbeleuchtung oder Direkteinkopplung selektiv ausgewählt und dargestellt werden. Bei dem RGB Bild 17b handelt es sich auch hier vorzugsweise um eine fotographische Darstellung des erfassten Hautbereichs 2, während das überlagerte Hyperspektralbild 17a eine Darstellung mit einer Vielzahl von Farbkanälen aufweist. Diese sind auch hier vorzugsweise selektiv mittels zugehörigen Einstellmitteln 18,18a auswählbar. Zudem kann auch hier eine Anzeige bestimmter Wellenlängen oder Strukturen wie beispielsweise bestimmten Spektren für Hämoglobin, Desoxyhämoglobin, Melanin etc. selektiv erfolgen.

Neben der überlagerten Darstellung 17a,17b in der Abbildung 21 kann analog zum Ausführungsbeispiel gemäß **Fig. 5a** ein berechneter Vorcharakterisierungs- oder Bewertungswert 19a dargestellt werden.

Die oben beschriebenen Ausführungsformen sind lediglich beispielhaft, wobei die Erfindung keineswegs auf die in den Figuren gezeigten Ausführungsformen beschränkt ist.

## Patentansprüche

1. Vorrichtung (10) zur Erzeugung einer Mehrzahl von Abbildungen zur Unterstützung bei der Charakterisierung von Hautläsionen, aufweisend eine Beleuchtungseinheit (1) zur selektiven Auflichtbeleuchtung (5b) eines Hautbereichs (2) und Direkteinkopplung von Licht (5a) in den Hautbereich (2), wobei die Beleuchtungseinheit eine Vielzahl von vorzugsweise gleichartigen LED Chips (3a,3b) aufweist,
eine Messeinheit (4) zur Auswertung einer Rückstreuung (5c) des von der Beleuchtungseinheit (1) beleuchteten Hautbereichs (2), wobei die Messeinheit (4) einen ersten Sensor (4a) zur Detektion von multispektralen oder hyperspektralen Bildinformationen und einen zweiten Sensor (4b) zur Detektion von RGB-Bildinformationen aufweist,
eine Steuereinheit (9) zur Ansteuerung der Beleuchtungseinheit (1) und der Messeinheit (4), und
eine Verarbeitungs- und Ausgabeeinheit (9a) zur Abbildungserzeugung basierend auf den von der Messeinheit (4) detektierten Bildinformationen, **dadurch gekennzeichnet, dass** die Beleuchtungseinheit (1) einen ersten Hautbeleuchtungsbereich (1a) an einem Endabschnitt (6a) der Beleuchtungseinheit (1) und einen davon beabstandeten zweiten Auflichtbeleuchtungsbereich (1b) aufweist, in welchem vorzugsweise jeweils eine Vielzahl von LED Chips (3a,3b) angeordnet ist, und wobei die Steuereinheit (9) ausgebildet ist, die Beleuchtungseinheit (1) und die Messeinheit (4) zur sequentiellen Detektion von Bildinformationen mittels Auflichtbeleuchtung (5b) und Direkteinkopplung von Licht (5a) anzusteuern..

2. Vorrichtung nach Anspruch 1.
wobei die LED Chips (3a,3b) in einem kontinuierlichen Spektrum emittieren und/oder wobei die Beleuchtungseinheit (1) nur LED Chips (3a,3b) aufweist, welche in einem im Wesentlichen gleichartigen Spektrum emittieren.

3. Vorrichtung nach Anspruch 1 oder 2,
wobei der Hautbeleuchtungsbereich (1a) und/oder der Auflichtbeleuchtungsbereich (1b) ringförmig oder rechteckförmig um einen Erfassungsbereich (6c) der Beleuchtungseinheit (1) angeordnet ist.

4. Vorrichtung nach einem der vorhergehenden Ansprüche,
wobei der erste Sensor (4a) ein multispektraler Sensor zur Detektion von elektromagnetischer Strahlung in wenigstens 4-11 vordefinierten Wellenlängenbereichen oder ein hyperspektraler Sensor zur Detektion von elektromagnetischer Strahlung in wenigstens 12- bis 20 vordefinierten Wellenlängenbereichen ist.

5. Vorrichtung nach einem der vorhergehenden Ansprüche,
wobei die Messeinheit (4) einen Strahlenteiler (8), vorzugsweise in der Form eines Strahlenteilerwürfels, aufweist, welcher die Rückstreuung (5c) an den ersten und den zweiten Sensor (4a,4b) mit vorzugsweise gleicher Intensität bereitstellt.

6. Vorrichtung nach einem der vorhergehenden Ansprüche,
wobei die Verarbeitungs- und Ausgabeeinheit (9a) ausgebildet ist, die erzeugten Abbildungen selektiv als Kombination von Abbildungen basierend auf RGB Bildinformationen und multi- oder hyperspektralen Bildinformation unter Auflichtbeleuchtung, als Kombination von Abbildungen basierend auf RGB Bildinformationen und multi- oder hyperspektralen Bildinformationen unter Direkteinkopplung der Beleuchtung und/oder als eine Abbildung einer wellenlängenabhängigen Intensitätscharakteristik in vordefinierten Spektralbereichen, vorzugsweise zwischen 400nm bis 1000nm, auszugeben.

7. Vorrichtung nach einem der vorhergehenden Ansprüche,
wobei die Verarbeitungs- und Ausgabeeinheit (9a) dazu ausgebildet ist, eine Vorcharakterisierung und/oder Bewertung der von der Messeinheit (4) erfassten Bildinformationen durchzuführen.

8. Vorrichtung nach Anspruch 7,
wobei die Verarbeitungs- und Ausgabeeinheit (9a) einen Algorithmus aufweist, welcher eine Analyse der von der Messeinheit (4) detektierten Bildinformationen mittels künstlicher Intelligenz, vorzugsweise mittels eines Deep-Learning Algorithmus, durchführt.

9. Vorrichtung nach einem der vorhergehenden Ansprüche,
wobei die Vorrichtung (10) ein mobiles Handgerät (10a), aufweisend die Beleuchtungseinheit (1) und die Messeinheit (4), und eine damit verbundene Recheneinheit (10b), vorzugsweise einen Rechner, Laptop oder Tablet, aufweisend die Verarbeitungs- und Ausgabeeinheit (9a) der Vorrichtung (10), umfasst.

10. Verfahren zur Erzeugung einer Mehrzahl von Abbildungen zur Unterstützung bei der Charakterisierung von Hautläsionen, vorzugsweise unter Verwendung einer Vorrichtung (10) nach einem der Ansprüche 1-11, aufweisend die Schritte:
Beleuchtung eines zu untersuchenden Hautbereichs (2) durch eine Beleuchtungseinheit (1) mittels einer Auflichtbeleuchtung (5b) und Direkteinkopplung (5a) von Licht in den Hautbereich (2) durch eine Vielzahl von vorzugsweise gleichartigen LED Chips (3a,3b),
Erfassung einer Rückstreuung (5c) des zu untersuchenden Hautbereichs (2) für die jeweilige Beleuchtung (5a,5b),
Detektion von multispektralen oder hyperspektralen Bildinformationen in der Rückstreuung (5c) mittels eines ersten Sensors (4a) einer Messeinheit (4),
Detektion von RGB Bildinformationen in der Rückstreuung (5c) mittels eines zweiten Sensors (4b) einer Messeinheit (4), und
Verarbeitung der detektierten Bildinformationen zur Erzeugung und Ausgabe von Abbildungen, **dadurch gekennzeichnet, dass** die Auflichtbeleuchtung (5b) und die Direkteinkopplung (5a) von Licht mittels zwei in unterschiedlicher Distanz zum zu untersuchenden Hautbereich (2) angeordneten Lichtquellenebenen (1a,1b) aufweisend jeweils eine Vielzahl von LED Chips (3a,3b) erfolgt, und wobei die Beleuchtungseinheit (1) und die Messeinheit (4) zur sequentiellen Detektion von Bildinformationen mittels Auflichtbeleuchtung (5b) und Direkteinkopplung von Licht (5a) durch eine Steuereinheit (9) angesteuert werden.

11. Verfahren nach Anspruch 10,
wobei die Detektion der multispektralen oder hyperspektralen Bildinformationen und die Detektion von RGB Bildinformationen für die jeweilige Beleuchtung (5a,5b) gleichzeitig erfolgen.

12. Verfahren nach Anspruch 10 oder 11,
wobei aus den multispektralen oder hyperspektralen Bildinformationen eine multispektrale oder hyperspektrale Abbildung mit einer Vielzahl selektiv darstellbarer Farbkanäle erzeugt wird und/oder wobei aus den RGB Bildinformationen eine RGB Abbildung mit drei selektiv einzeln oder kombiniert darstellbaren Farbkanälen erzeugt wird.

13. Verfahren nach einem der Ansprüche 10 bis 12,
wobei eine Ausgabe selektiv als Kombination von erzeugten Abbildungen basierend auf RGB Bildinformationen und multi- oder hyperspektralen Bildinformation unter Auflichtbeleuchtung, als Kombination von erzeugten Abbildungen basierend auf RGB Bildinformationen und multi- oder hyperspektralen Bildinformationen unter Direkteinkopplung der Beleuchtung und/oder als Abbildung einer wellenlängenabhängige Intensitätscharakteristik in vordefinierten Spektralbereichen, vorzugsweise zwischen 400nm bis 1000nm, erfolgt.

## Claims

1. A device (10) for generating a plurality of images for supporting the characterization of skin lesions, the device comprising
an illumination unit (1) for selective incident illumination (5b) of an area of skin (2) and direct coupling in of light (5a) into the area of skin (2), the illumination unit comprising a plurality of preferably similar LED chips (3a, 3b),
a measuring unit (4) for evaluating backscatter (5c) of the area of skin (2) illuminated by the illumination unit (1), the measuring unit (4) comprising a first sensor (4a) for detecting multispectral or hyperspectral image information and a second sensor (4b) for detecting RGB image information,
a control unit (9) for controlling the illumination unit (1) and the measuring unit (4), and
a processing and output unit (9a) for image generation based on the image information detected by the measuring unit (4),
**characterized in that**
the illumination unit (1) has a first skin illumination area (1a) at an end portion (6a) of the illumination unit (1) and a second incident illumination area (1b) spaced apart therefrom, in each of which a plurality of LED chips (3a, 3b) are preferably disposed, and the control unit (9) is configured to control the illumination unit (1) and the measuring unit (4) to sequentially detect image information by incident illumination (5b) and direct coupling in of light (5a).

2. The device according to claim 1,
wherein the LED chips (3a, 3b) emit light in a continuous spectrum and/or wherein
the illumination unit (1) comprises only LED chips (3a, 3b) that emit light in an essentially similar spectrum.

3. The device according to claim 1 or 2,
wherein the skin illumination area (1a) and/or the incident illumination area (1b) are disposed in the shape of a ring or a rectangle around a detection area (6c) of the illumination unit (1).

4. The device according to any one of the preceding claims,
wherein the first sensor (4a) is a multispectral sensor for detecting electromagnetic radiation in at least 4 to 11 predefined wavelength ranges or a hyperspectral sensor for detecting electromagnetic radiation in at least 12 to 20 predefined wavelength ranges.

5. The device according to any one of the preceding claims,
wherein the measuring unit (4) comprises a beam splitter (8), preferably in the form of a beam splitter cube, which provides the backscatter (5c) to the first and second sensors (4a, 4b) at preferably the same intensity.

6. The device according to any one of the preceding claims,
wherein the processing and output unit (9a) is configured to output the generated images selectively as a combination of images based on RGB image information and multispectral or hyperspectral image information under incident illumination, as a combination of images based on RGB image information and multispectral or hyperspectral image information under direct coupling in of the illumination, and/or as an image of a wavelength-dependent intensity characteristic in predefined spectral ranges, preferably between 400 nm to 1000 nm.

7. The device according to any one of the preceding claims,
wherein the processing and output unit (9a) is configured to pre-characterize and/or assess the image information acquired by the measuring unit (4).

8. The device according to claim 7,
wherein the processing and output unit (9a) comprises an algorithm which analyzes the image information detected by the measuring unit (4) by means of artificial intelligence, preferably by means of a deep learning algorithm.

9. The device according to any one of the preceding claims,
wherein the device (10) comprises a mobile hand-held device (10a), which comprises the illumination unit (1) and the measuring unit (4), and a computing unit (10b), preferably a computer, a laptop or a tablet, which is connected thereto and comprises the processing and output unit (9a) of the device (10).

10. A method for generating a plurality of images for supporting the characterization of skin lesions, preferably using a device (10) according to any one of claims 1 to 11, the method comprising the following steps:
illuminating an area of skin (2) to be examined by incident illumination (5b) and direct coupling in (5a) of light into the area of skin (2) by means of a plurality of preferably similar LED chips (3a, 3b) using an illumination unit (1),
detecting backscatter (5c) of the area of skin (2) to be examined for the respective illuminations (5a, 5b),
detecting multispectral or hyperspectral image information in the backscatter (5c) by means of a first sensor (4a) of a measuring unit (4),
detecting RGB image information in the backscatter (5c) by means of a second sensor (4b) of a measuring unit (4), and
processing the detected image information to generate and output images,
**characterized in that**
the incident illumination (5b) and the direction coupling in (5a) of light takes place by means of two light source planes (1a, 1b) disposed at different distances from the area of skin (2) to be examined and each comprising a plurality of LED chips (3a, 3b), and the illumination unit (1) and the measuring unit (4) are controlled by a control unit (9) to sequentially detect image information by incident illumination (5b) and direct coupling in of light (5a).

11. The method according to claim 10,
wherein the detection of the multispectral or hyperspectral image information and the detection of RGB image information for the respective illuminations (5a, 5b) take place simultaneously.

12. The method according to claim 10 or 11,
wherein a multispectral or hyperspectral image with a plurality of selectively displayable color channels is generated from the multispectral or hyperspectral image information, and/or wherein an RGB image with three color channels selectively displayable individually or in combination is generated from the RGB image information.

13. The method according to any one of claims 10 to 12,
wherein an output is selectively in the form of a combination of generated images based on RGB image information and multispectral or hyperspectral image information under incident illumination, in the form of a combination of generated images based on RGB image information and multispectral or hyperspectral image information under direct coupling in of the illumination, and/or in the form of an image of a wavelength-dependent intensity characteristic in predefined spectral ranges, preferably between 400 nm to 1000 nm.

## Revendications

1. Dispositif (10) pour la génération d'une pluralité d'images destinées à supporter la caractérisation de lésions cutanées, le dispositif comprenant :
une unité d'éclairage (1) pour l'éclairage incident (5b) sélectif d'une région de peau (2) et l'introduction directe de lumière (5a) dans la région de peau (2), l'unité d'éclairage comprenant une pluralité de puces DEL (3a, 3b) de préférence similaires,
une unité de mesure (4) destinée à évaluer une rétrodiffusion (5c) de la région de peau (2) éclairée par l'unité d'éclairage (1), l'unité de mesure (4) comprenant un premier capteur (4a) destiné à détecter des informations d'image multispectrales ou hyperspectrales et un deuxième capteur (4b) destiné à détecter des informations d'image RVB,
une unité de commande (9) destinée à commander l'unité d'éclairage (1) et l'unité de mesure (4) et
une unité de traitement et de sortie (9a) destinée à générer des images sur la base des informations d'image détectées par l'unité de mesure (4),
**caractérisé en ce que**
l'unité d'éclairage (1) a une première zone d'éclairage de peau (1a) à une partie d'extrémité (6a) de l'unité d'éclairage (1) et une deuxième zone d'éclairage incident (1b) espacée de celle-ci, dans chacune desquelles une pluralité de puces DEL (3a, 3b) sont de préférence disposées, et l'unité de commande (9) est configurée pour commander l'unité d'éclairage (1) et l'unité de mesure (4) à détecter séquentiellement des informations d'image par éclairage incident (5b) et par introduction directe de lumière (5a).

2. Dispositif selon la revendication 1,
dans lequel les puces DEL (3a, 3b) émettent de la lumière dans un spectre continu et/ou dans lequel l'unité d'éclairage (1) ne comprend que des puces DEL (3a, 3b) qui émettent de la lumière dans un spectre essentiellement similaire.

3. Dispositif selon la revendication 1 ou 2,
dans lequel la zone d'éclairage de peau (la) et/ou la zone d'éclairage incident (1b) sont disposées de manière annulaire ou rectangulaire autour d'une zone de détection (6c) de l'unité d'éclairage (1).

4. Dispositif selon l'une quelconque des revendications précédentes,
dans lequel le premier capteur (4a) est un capteur multispectral destiné à détecter du rayonnement électromagnétique dans au moins 4 à 11 intervalles de longueur d'onde prédéfinis ou un capteur hyperspectral destiné à détecter du rayonnement électromagnétique dans au moins 12 à 20 intervalles de longueur d'onde prédéfinis.

5. Dispositif selon l'une quelconque des revendications précédentes,
dans lequel l'unité de mesure (4) comprend un séparateur de faisceau (8), de préférence en forme de cube séparateur de faisceau, qui fournit la rétrodiffusion (5c) au premier capteur (4a) et au deuxième capteur (4b) de préférence à la même intensité.

6. Dispositif selon l'une quelconque des revendications précédentes,
dans lequel l'unité de traitement et de sortie (9a) est configurée pour fournir sélectivement les images générées comme combinaison d'images sur la base d'informations d'image RVB et d'informations d'image multispectrales ou hyperspectrales sous éclairage incident, comme combinaison d'images sur la base d'informations d'image RVB et d'informations d'image multispectrales ou hyperspectrales sous introduction directe de l'éclairage et/ou comme image d'une caractéristique d'intensité dépendante de la longueur d'onde dans des intervalles de spectre prédéfinis, de préférence entre 400 nm à 1000 nm.

7. Dispositif selon l'une quelconque des revendications précédentes,
dans lequel l'unité de traitement et de sortie (9a) est configurée pour précaractériser et/ou évaluer les informations d'image acquises par l'unité de mesure (4).

8. Dispositif selon la revendication 7,
dans lequel l'unité de traitement et de sortie (9a) comprend un algorithme qui analyse les informations d'image détectées par l'unité de mesure (4) au moyen de l'intelligence artificielle, de préférence au moyen d'un algorithme d'apprentissage profond.

9. Dispositif selon l'une quelconque des revendications précédentes,
dans lequel le dispositif (10) comprend un appareil portatif (10a) mobile, qui comprend l'unité d'éclairage (1) et l'unité de mesure (4), et une unité de calcul (10b), de préférence un ordinateur, un laptop ou une tablette, qui est reliée à celui-ci et qui comprend l'unité de traitement et de sortie (9a) du dispositif (10).

10. Procédé pour la génération d'une pluralité d'images destinées à supporter la caractérisation de lésions cutanées, de préférence en utilisant un dispositif (10) selon l'une quelconque des revendications 1 à 11, le procédé comprenant les étapes consistant à :
éclairer une région de peau (2) à examiner par éclairage incident (5b) et par introduction directe (5a) de la lumière dans la région de peau (2) au moyen d'une pluralité de puces DEL (3a, 3b) de préférence similaires en utilisant une unité d'éclairage (1),
détecter de la rétrodiffusion (5c) de la région de peau (2) à examiner pour les éclairages respectifs (5a, 5b),
détecter des informations d'image multispectrales ou hyperspectrales dans la rétrodiffusion (5c) au moyen d'un premier capteur (4a) d'une unité de mesure (4),
détecter des informations d'image RVB dans la rétrodiffusion (5c) au moyen d'un deuxième capteur (4b) d'une unité de mesure (4) et
traiter les informations d'image détectées afin de générer et fournir des images,
**caractérisé en ce que**
l'éclairage incident (5b) et l'introduction directe (5a) de lumière s'effectuent au moyen de deux plans de source de lumière (1a, 1b) disposés à des distances différentes de la région de peau (2) à examiner et comprenant chacun une pluralité de puces DEL (3a, 3b), et l'unité d'éclairage (1) et l'unité de mesure (4) sont commandées par une unité de commande (9) à détecter séquentiellement des informations d'image par éclairage incident (5b) et par introduction directe de lumière (5a).

11. Procédé selon la revendication 10,
dans lequel la détection des informations d'image multispectrales ou hyperspectrales et la détection d'informations d'image RVB pour les éclairages (5a, 5b) respectifs s'effectuent simultanément.

12. Procédé selon la revendication 10 ou 11,
dans lequel une image multispectrale ou hyperspectrale avec une pluralité de canaux de couleur affichables par choix est générée à partir des informations d'image multispectrales ou hyperspectrales et/ou dans lequel une image RVB avec trois canaux de couleur affichables individuellement ou en combinaison par choix est générée à partir des informations d'image RVB.

13. Procédé selon l'une quelconque des revendications 10 à 12,
dans lequel une sortie s'effectue par choix comme combinaison d'images générées sur la base d'informations d'image RVB et d'informations d'image multispectrales ou hyperspectrales sous éclairage incident, comme combinaison d'images générées sur la base d'informations d'image RVB et d'informations d'image multispectrales ou hyperspectrales sous introduction directe de l'éclairage et/ou comme image d'une caractéristique d'intensité dépendante de la longueur d'onde dans des intervalles de spectre prédéfinis, de préférence entre 400 nm à 1000 nm.
